# FASCICULE DE BREVET EUROPEEN

(11) **EP 3 361 929 B1**
(45) Date de publication et mention de la délivrance du brevet: **02.12.2020**
(21) Numéro de dépôt: 16795119.3
(22) Date de dépôt: 14.10.2016
(51) Int. Cl.: A61B 3/024, A61B 3/032, A61B 3/113, G02C 13/00

(54) **DISPOSITIF DE TEST DU COMPORTEMENT VISUEL D'UN INDIVIDU ET MÉTHODE DE DÉTERMINATION D'AU MOINS UN PARAMÈTRE DE CONCEPTION OPTIQUE D'UNE LENTILLE OPHTALMIQUE UTILISANT UN TEL DISPOSITIF**
VORRICHTUNG ZUR PRÜFUNG DES VISUELLEN VERHALTENS EINER PERSON UND VERFAHREN ZUR BESTIMMUNG VON ZUMINDEST EINEM OPTISCHEN DESIGNPARAMETERS EINES BRILLENGLASES MIT SOLCH EINER VORRICHTUNG
DEVICE FOR TESTING THE VISUAL BEHAVIOR OF A PERSON, AND METHOD FOR DETERMINING AT LEAST ONE OPTICAL DESIGN PARAMETER OF AN OPHTHALMIC LENS USING SUCH A DEVICE

(30) Priorité: 15.10.2015 FR 1559812
(43) Date de publication de la demande: 22.08.2018
(73) Titulaire: Essilor International, 94220 Charenton-Le-Pont (FR)
(72) Inventeur: ESCALIER, Guilhem, 94220 Charenton-Le-Pont (FR); POULAIN, Isabelle, 94220 Charenton-Le-Pont (FR); ROUSSEAU, Benjamin, 94220 Charenton-Le-Pont (FR)
(74) Mandataire: Jacobacci Coralis Harle
(86) Numéro de dépôt international: PCT/FR2016/052665
(87) Numéro de publication internationale: WO 2017/064441

(56) Documents cités:
- WO-A1-99/22638
- WO-A1-2008/139137
- US-A1- 2013 314 668
- US-A1- 2014 171 756
- US-A1- 2015 051 508

## Description

### DOMAINE TECHNIQUE AUQUEL SE RAPPORTE L'INVENTION

La présente invention concerne de manière générale un dispositif de test permettant de déterminer le comportement visuel d'un individu.

Elle concerne également une méthode de détermination d'au moins un paramètre de conception optique d'une lentille ophtalmique utilisant un tel dispositif.

### ARRIERE-PLAN TECHNOLOGIQUE

La personnalisation toujours plus précise des lentilles ophtalmiques d'une monture destinée à équiper un individu pour corriger sa vision nécessite une connaissance accrue du comportement visuel de l'individu dans des conditions de vision qui soient naturelles et représentatives de l'utilisation réelle desdites lentilles ophtalmiques.

La détermination de paramètres du comportement visuel de l'individu permet alors d'améliorer la conception optique des lentilles ophtalmiques qui seront montées dans la monture.

En particulier, lors de la conception optique des lentilles ophtalmiques progressives, il est particulièrement important de disposer de données de conception optique qui soient pertinentes pour rendre compte de l'utilisation de ces lentilles en vision de près et de la posture prise par l'individu, notamment en situation de lecture.

Cependant, les mesures effectuées actuellement par l'opticien sur l'individu sont le plus souvent contraintes, notamment du fait que l'individu ne porte pas de correction ou que les appareils de mesure ou dispositifs de test visuel utilisés placent l'individu dans une posture non naturelle, en particulier en vision de près.

Il en est de même lorsque l'individu porte une correction, qui, de plus, peut ne plus être adaptée à sa réfraction. Dans ce cas, les mesures sont faussées car la puissance optique impacte la posture à cause de l'effet prismatique de la correction.

De plus, il est difficile de mettre l'individu en situation de lecture dans une posture naturelle, sans que celui-ci soit équipé de son équipement de correction visuelle, les individus souffrant de myopie ayant tendance à réduire la distance de lecture, alors que ceux souffrant de presbytie ont tendance à augmenter cette distance.

Inversement, une mesure faite en demandant à l'individu de réaliser une tâche visuelle autre que de la lecture s'éloigne des conditions naturelles et donc d'une mesure représentative de l'utilisation d'un verre progressif au quotidien.

Les dispositifs de test visuel sont donc peu précis et les paramètres de conception optique déterminés par l'opticien ne sont donc pas toujours représentatifs du comportement visuel de l'individu en conditions naturelles.

Il en résulte que la conception optique des lentilles progressives n'est pas optimale de sorte que l'adaptation des lentilles ophtalmiques progressives au porteur peut être difficile.

Le document WO 2008/139137 décrit un dispositif de test du comportement visuel d'un individu selon la préambule de la revendication 1.

### OBJET DE L'INVENTION

Afin de remédier à l'inconvénient précité de l'état de la technique, la présente invention propose un dispositif de test du comportement visuel d'un individu permettant de déterminer de manière simple et précise le comportement visuel d'un individu.

Plus particulièrement, on propose selon l'invention un dispositif de test du comportement visuel d'un individu comportant :
- un afficheur actif adapté à afficher au moins une cible visuellement prépondérante en une pluralité de positions variables au cours du temps et alignées selon au moins une ligne ou une colonne, et
- une unité de commande de l'afficheur, programmée pour que les positions d'affichage successives de la cible suivent, au cours du temps, un protocole de suivi visuel.

Ainsi, grâce au dispositif selon l'invention, il est possible de tester le comportement visuel de l'individu dans une situation de lecture en conditions naturelles. Ladite cible peut être suivie du regard par tout individu, adulte ou enfant, sachant lire ou non, et de manière indépendante de la langue parlée par l'individu. De plus, la cible peut être suivie du regard même en l'absence de correction visuelle portée par l'individu.

On peut prévoir en particulier que toutes les positions d'affichage successives soient telles que la cible est dans le champ visuel de l'individu. Autrement dit, l'individu peut suivre la cible tout au long du test visuel.

Ainsi, il n'y a pas de risque qu'une cible, visuellement prépondérante ou pas, ne soit pas détectée par le dispositif de test.

Au contraire, le dispositif de test est conçu pour imposer à l'individu de regarder dans des directions bien particulières de son champ visuel, et en aucune façon en dehors de celui-ci, au risque d'introduire des risques d'erreur de mesure.

Grâce au dispositif selon l'invention, un paramètre de comportement visuel précis de l'individu peut être déterminé.

Selon une caractéristique particulièrement avantageuse de l'invention, lesdites positions de la cible sont alignées selon au moins deux lignes ou deux colonnes sensiblement parallèles.

Avantageusement encore, ladite pluralité de positions comprend, sur chaque ligne ou colonne, au moins trois positions alignées de ladite cible.

D'autres caractéristiques non limitatives et avantageuses de la méthode conforme à l'invention, prises individuellement ou selon toutes les combinaisons techniquement possibles, sont les suivantes :
- l'unité de commande impose, dans chaque position dudit protocole de suivi visuel, que la cible s'affiche pendant une durée prédéterminée ;
- ladite durée prédéterminée est comprise entre 50 millisecondes et 1 seconde ;
- ladite cible est fixe pendant ladite durée prédéterminée ;
- l'unité de commande impose un délai prédéterminé entre les affichages de la cible dans deux positions successives du protocole de suivi visuel ;
- ledit délai prédéterminé varie au cours du protocole de suivi visuel ;
- ladite cible est invisible pendant ledit délai prédéterminé ;
- ladite cible est visible pendant ledit délai prédéterminé et se déplace entre les deux positions successives correspondantes du protocole de suivi visuel, de l'une à l'autre ;
- ladite unité de commande impose que deux positions successives du protocole de suivi visuel sont séparées d'une distance inférieure à 10 centimètres ;
- ladite unité de commande impose que deux positions successives du protocole de suivi visuel sont séparées d'une distance variant le long du protocole de suivi visuel ;
- ladite unité de commande mémorise un sens de parcours vertical et horizontal privilégié du protocole de suivi visuel ;
- l'affichage de ladite cible dans deux positions successives du protocole de suivi visuel suit ledit sens de parcours privilégié au moins six fois sur dix ;
- lesdites lignes sensiblement parallèles le long desquelles sont alignées les positions prédéterminées de la cible s'étendant sensiblement horizontalement, le sens de parcours du protocole de suivi visuel est identique pour toutes les lignes prises successivement de la plus haute à la plus basse, de droite à gauche ou de gauche à droite ;
- lesdites lignes sensiblement parallèles le long desquelles sont alignées les positions prédéterminées de la cible s'étendant sensiblement verticalement, le sens de parcours du protocole de suivi visuel est identique, de haut en bas ou de bas en haut, pour toutes les lignes prises successivement de gauche à droite ou de droite à gauche ;
- l'unité de commande est programmée pour permettre la sélection dudit protocole de suivi visuel parmi une pluralité de protocoles de suivi visuel enregistrée dans une base de données locale ou distante, dans laquelle un sens de parcours est enregistré en association avec le protocole de suivi visuel auquel il correspond ;
- le protocole de suivi visuel suit un trajet de lecture conforme à celui défini par un système d'écriture donné, de manière à reproduire le déplacement du regard de l'individu pendant la lecture conformément au système d'écriture.

L'invention propose également une méthode de détermination d'au moins un paramètre de conception optique d'une lentille ophtalmique destinée à équiper une monture choisie par un individu, en fonction du comportement visuel de celui-ci.

Selon l'invention, cette méthode utilise le dispositif de test précité et comprend les étapes suivantes :
a) on demande à l'individu d'effectuer une tâche visuelle dans laquelle il regarde la cible affichée par ledit afficheur du dispositif d'affichage, les positions de ladite cible étant prédéterminées dans un référentiel lié à un appareil de capture d'image,
b) on capture, au moyen dudit appareil de capture d'image, des images de la tête de l'individu regardant ladite cible, chaque image correspondant à une position prédéterminée de ladite cible,
c) on détermine, à partir d'une partie au moins des images de la tête de l'individu, des positions de la tête de l'individu dans un référentiel lié audit appareil de capture d'image ou des directions de regard de l'individu dans un référentiel lié à la tête de l'individu, chaque position de la tête ou direction de regard de l'individu étant associée à la position de ladite cible pour laquelle l'image correspondante de la tête de l'individu a été capturée,
d) on déduit ledit paramètre de conception optique recherché à partir desdites positions de tête ou direction de regard de l'individu.

La méthode selon l'invention permet de déterminer un paramètre de conception optique qui soit représentatif du comportement visuel de l'individu.

La détermination du paramètre de conception optique grâce à cette méthode permet ainsi une meilleure adaptation des zones de vision de la lentille ophtalmique, en fonction des caractéristiques de l'individu.

Selon une caractéristique particulièrement avantageuse de l'invention, préalablement à l'étape d), on réexprime, à partir desdites positions déterminées de la tête ou des directions de regard de l'individu, les positions de ladite cible dans un référentiel lié à la tête de l'individu, et à l'étape d), on déduit ledit paramètre de conception optique recherché à partir desdites positions de la cible dans le référentiel lié à la tête de l'individu.

### DESCRIPTION DETAILLEE D'UN EXEMPLE DE REALISATION

La description qui va suivre en regard des dessins annexés, donnés à titre d'exemples non limitatifs, fera bien comprendre en quoi consiste l'invention et comment elle peut être réalisée.

Sur les dessins annexés :
- la figure 1 est une vue schématique d'un individu tenant dans ses mains un dispositif de test conforme à l'invention ;
- la figure 2 est une vue de face du dispositif de test de la figure 1 ;
- la figure 3 est une vue schématique rassemblant différents protocoles de suivi visuel utilisés dans le dispositif de la figure 1 ;
- la figure 4 représente un diagramme d'une méthode de détermination d'un paramètre de conception optique utilisant le dispositif de la figure 1 ;
- la figure 5 est une vue schématique de la tête de l'individu ;
- la figure 6 représente un repère lié à la tête de l'individu ;
- les figures 7 à 11 représentent le dispositif de test de la figure 1 lors de différentes étapes d'une phase de réglage de la méthode de l'invention ;
- la figure 12 illustre un repère lié au dispositif de test de la figure 1 ;
- la figure 13 représente le dispositif de test de la figure 1 lors d'une étape d'une phase d'entraînement de la méthode de l'invention ;
- les figures 14 et 15 représentent le dispositif de test lors d'une étape de vérification d'une phase de mesure de la méthode de l'invention ;
- la figure 16 représente l'afficheur du dispositif de test avec des cibles affichées et un repère lié à la tête de l'individu regardant la cible dans une position finale du protocole ;
- les figures 17 et 18 représentent des exemples de positions mesurées de la cible dans le référentiel lié à la tête de l'individu au cours du protocole de lecture.

En préambule, on notera que les éléments identiques ou similaires des différents modes de réalisation représentés sur les différentes figures seront référencés par les mêmes signes de référence et ne seront pas décrits à chaque fois.

On notera également que dans l'exposé qui va suivre, les termes « haut » (ou « supérieur ») et « bas » (ou « inférieur ») seront utilisés relativement à l'individu utilisant le dispositif de test, le haut désignant le côté tourné vers la tête de l'individu et le bas désignant le côté tourné vers les pieds de l'individu.

De même, le terme « avant » désignera le côté tourné vers l'individu, le terme « arrière » désignant le côté opposé au côté avant.

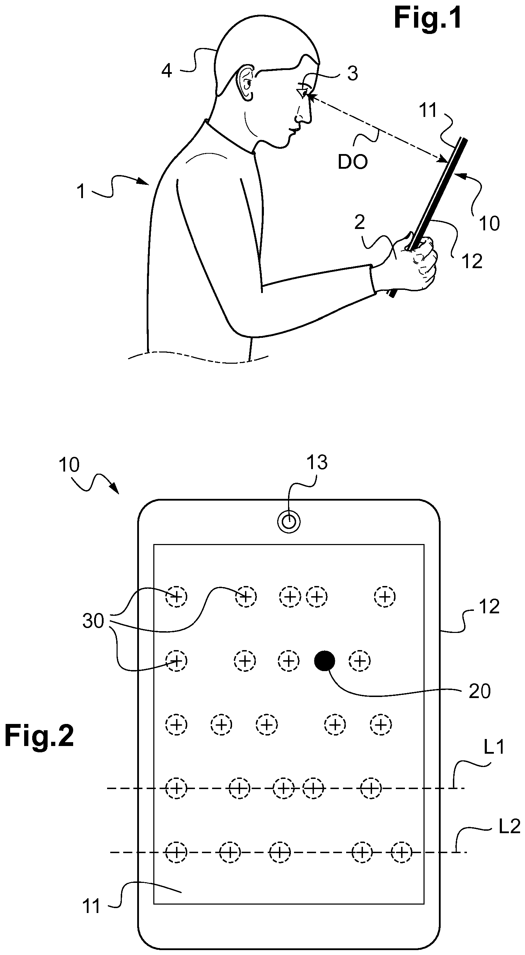

Sur la figure 1, on a représenté un individu 1 dont on souhaite tester le comportement visuel.

À cet effet, l'individu 1 tient dans ses mains 2 un dispositif de test 10 conforme à l'invention destiné à déterminer ce comportement visuel dans des conditions données.

Plus particulièrement ici, on souhaite utiliser le dispositif de test 10 pour analyser de manière générale la vision de près de l'individu 1, et en particulier le comportement visuel qu'il adopte lorsqu'il est en situation de lecture.

On considérera que la vision de près correspond à une distance d'observation DO (voir figure 1) entre l'œil 3 de l'individu 1 et le dispositif de test 10 inférieure à 70 centimètres (cm).

Dans d'autres modes de réalisation, la vision intermédiaire (DO comprise entre 70 cm et 4 mètres) ou la vision de loin (DO supérieure à 4 m) peuvent être testées grâce au dispositif de test selon l'invention.

Selon l'invention, le dispositif de test 10 comporte (voir figure 2) :
- un afficheur 11 actif qui affiche une cible 20 visuellement prépondérante en une pluralité de positions 30 variables au cours du temps et alignées selon au moins une ligne ou une colonne, et
- une unité de commande (non représentée) de l'afficheur 11, programmée pour que les positions 30 d'affichage successives de la cible 20 suivent, au cours du temps, un protocole de suivi visuel, dans lequel toutes les positions d'affichage successives sont telles que la cible est dans le champ visuel de l'individu.

L'afficheur 11 du dispositif de test peut afficher, à chaque instant du test visuel, une seule et unique cible ou bien plusieurs cibles simultanément, chaque cible étant dans le champ visuel de l'individu. Dans les deux cas, la cible visuellement prépondérante est celle qui est adaptée à accrocher le regard de l'individu et que l'individu va suivre au cours du test visuel.

Si celle-ci n'était pas dans le champ visuel de l'individu, celui-ci ne pourrait pas la détecter.

Lorsque plusieurs cibles sont affichées par l'afficheur 11 dans le champ visuel de l'individu, la cible visuellement prépondérante peut être, par exemple, une cible plus lumineuse ou plus contrastée, de couleur ou de forme (rond, carré, étoile, ...) différente, ou de taille plus petite ou plus grande que les autres, ou bien encore une cible qui clignote alors que les autres ne clignotent pas. Les différentes cibles affichées par l'afficheur peuvent également comprendre un ensemble d'indicateurs ou bien former une grille de points gris.

Dans les modes de réalisation où l'afficheur n'affiche qu'une seule cible, celle-ci peut prendre une pluralité de positions sur l'afficheur 11, dans la mesure où les positions prises restent dans le champ visuel de l'individu. Ces positions sont « variables » en ce sens que la cible se déplace séquentiellement d'une position à une autre au cours du test visuel. On notera néanmoins que la séquence des positions prises successivement par la cible dans ces modes de réalisation peut comprendre deux positions identiques. En d'autres termes, il est possible qu'au cours du test visuel la cible repasse par une position déjà prise précédemment.

Dans les modes de réalisation où l'afficheur affiche plusieurs cibles dont l'une est visuellement prépondérante, les positions d'affichage des cibles peuvent être variables au cours du temps, à l'intérieur du champ visuel de l'individu, mais en tout état de cause, la cible visuellement prépondérante est celle qui se déplace selon une séquence de positions dans le champ visuel de l'individu de manière à imposer à l'individu 1 une succession de directions de regard particulières.

Dans la présente description, on entendra donc par « *protocole de suivi visuel »* la séquence d'affichage de la cible 20 visuellement prépondérante au cours du test visuel réalisé par l'individu 1, ces positions d'affichage successives étant telles que la cible 20 est dans le champ visuel de l'individu 1.

En d'autres termes, ce protocole de suivi visuel correspond à la succession, dans le temps, des positions prises par la cible visuellement prépondérante. Grâce à celle-ci, on impose un protocole à l'individu qui regarde successivement dans une pluralité de directions particulières désirées qui sont chacune associées à une position particulière prise par la cible. De cette façon, si les positions de cette cible sont connues, il est alors possible, dans certaines conditions, de remonter à l'information concernant la direction de regard de l'individu lors du test visuel.

Comme illustré sur la figure 2, le dispositif de test 10 se présente ici sous la forme d'une tablette numérique. Cette tablette numérique comprend un écran qui constitue l'afficheur 11 du dispositif de test 10. Elle comprend également un boîtier 12 entourant l'écran et une caméra frontale 13 visant l'individu 1. L'unité de commande du dispositif 10 correspond, quant à elle, au contrôleur d'affichage de l'écran de la tablette qui est logé à l'intérieur du boîtier 12.

La cible 20 comprend ici un disque lumineux qui est affiché sur l'écran de la tablette, la taille de la cible étant suffisante pour qu'elle soit visible par l'individu 1 dans les conditions du test visuel. Ici, en conditions de lecture et en vision de près (DO < 70 cm), la cible 20 a une taille caractéristique (par ex. diamètre) supérieure à 5 millimètres.

De manière avantageuse, la taille caractéristique de la cible 20 est déterminée de telle sorte qu'elle puisse être vue avec une acuité supérieure à 0.1 dixième à 70 cm.

En variante, la cible peut comprendre un motif géométrique, régulier ou non. Il s'agit de préférence d'un motif quelconque, à l'exclusion d'un signe utilisé par un système d'écriture quelconque compréhensible par l'individu. En particulier, la cible visuellement prépondérante est dénuée de signification pour l'individu. Par exemple, la cible n'est pas un mot intelligible pour l'individu.

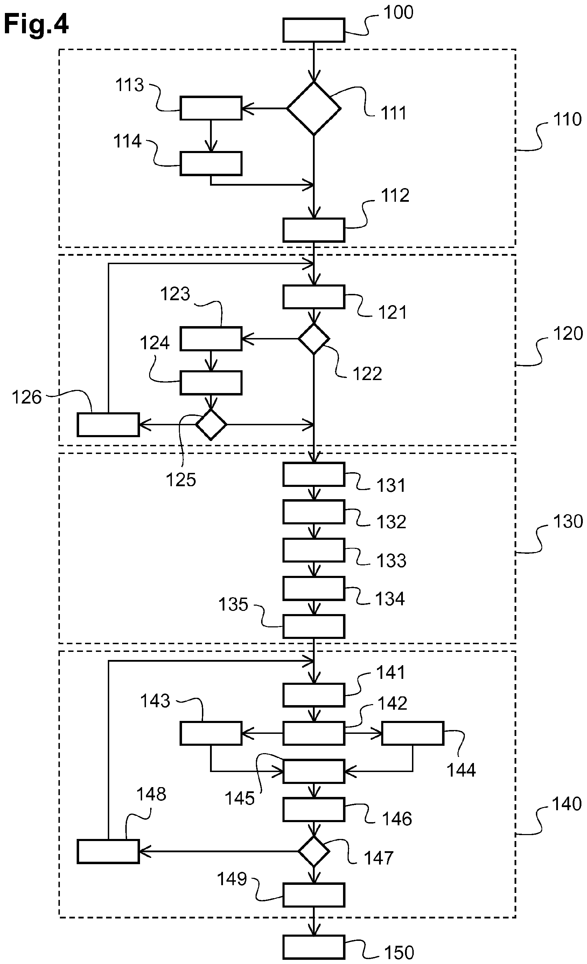

On va maintenant décrire, en référence à la figure 4, différents types de protocoles de suivi visuel qui sont mis en œuvre par le dispositif de test 10 selon l'invention et qui sont destinés ici à simuler la lecture d'un texte par l'individu 1.

De manière avantageuse, l'affichage de la cible selon les protocoles de suivi visuel mis en œuvre par le dispositif de test 10 constitue un stimulus visuel pour l'individu 1, destiné à lui faire bouger les yeux par suivi de cette cible 20 selon le même schéma que celui que l'individu 1 adopterait s'il lisait réellement un texte.

En d'autres termes, l'affichage de la cible visuellement prépondérante sur l'afficheur est commandé de telle sorte que, lorsque l'individu suit du regard la cible, la direction du regard de l'individu présente des directions de regard successives tout à fait similaires aux directions de regard qu'aurait cet individu en lisant un texte.

La séquence des positions d'affichage prises successivement par la cible visuellement prépondérante est de préférence prédéterminée en fonction d'un texte de référence, et/ou d'un modèle de lecture, correspondant aux caractéristiques et/ou aux préférences de lecture/écriture de l'individu.

Par exemple, la séquence peut être prédéterminée préalablement avec un autre dispositif, au cours d'une opération de calibration lors de laquelle on demande à l'individu de choisir un texte de référence, et/ou d'un modèle de lecture, parmi une pluralité de textes réels disponibles et de le lire à voix haute. La vitesse de lecture peut alors servir de paramètre pour la détermination des positions d'affichage de la cible.

La séquence peut également être prédéterminée en fonction de l'âge de l'individu ou en fonction d'un niveau de lecture déclaré par l'individu, à la suite d'un questionnaire rempli par l'individu.

On peut également envisager de faire un entraînement avec une vitesse moyenne, demander à l'individu si cette vitesse moyenne était trop ou pas assez rapide et ajuster la vitesse en fonction de sa réponse.

On observera tout d'abord que la lecture d'un texte par un individu se fait naturellement selon un schéma de lecture comportant trois opérations distinctes : fixations, saccades et rétro-saccades.

Lors des fixations, l'individu déchiffre le mot qu'il est en train de lire, c'est-à-dire le mot sur lequel le regard de l'individu est fixé.

Lors des saccades, correspondant aux phases de déplacement, c'est-à-dire aux passages de la lecture d'un mot au mot suivant, les yeux de l'individu bougent rapidement pour passer d'une fixation à une autre.

Ces saccades sont liées à l'empan visuel, c'est-à-dire au nombre de caractères (lettres, symboles, idéogrammes, etc...) qui sont déchiffrables pour une fixation donnée. Elles permettent au lecteur de déchiffrer tous les caractères d'un texte.

Les saccades se font généralement dans le sens de la lecture du texte. Néanmoins, les yeux effectuent également des « rétro-saccades » très rapides dans le sens opposé au sens de lecture pour passer d'une fixation à une autre. Ce mouvement est induit par une erreur des muscles oculomoteurs ou par une mauvaise lecture et compréhension du texte.

Un des avantages du dispositif de test 10 selon l'invention est de proposer des protocoles de suivi visuel qui se rapprochent le plus possible des schémas de lecture de l'individu.

Le dispositif de test 10 permet donc de simuler simplement la lecture d'un texte et de placer l'individu dans une situation où il adoptera une posture naturelle proche de celle qu'il adopterait pour une lecture en vision de près.

Une détermination du comportement visuel de l'individu dans ces conditions est donc rendue plus précise et la conception optique d'une lentille ophtalmique destinée à l'individu peut être améliorée afin que le design de la lentille ophtalmique réponde aux besoins de correction visuelle de l'individu.

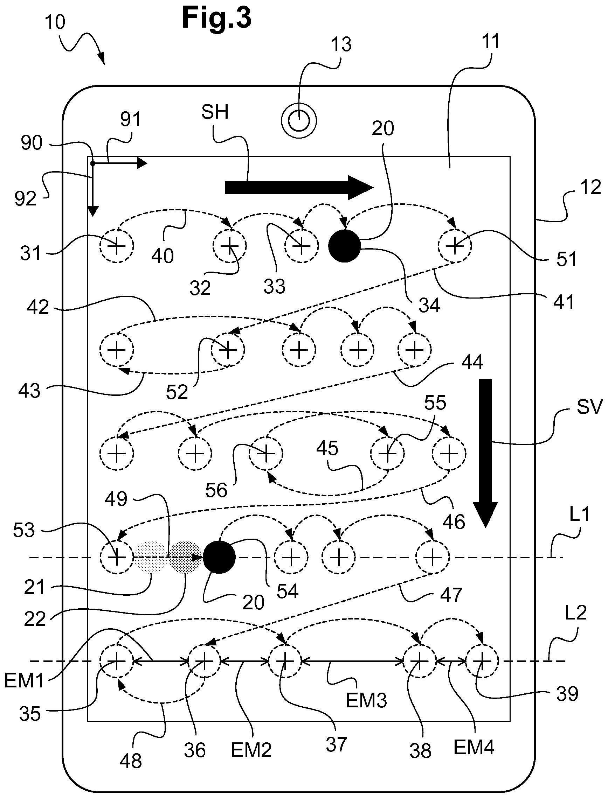

De préférence, les positions de la cible 20 sont alignées selon au moins deux lignes L1, L2 (cas des positions 35, 36, 37, 38, 39 pour la ligne L2 de la figure 3) sensiblement parallèles. Plus précisément, sur l'exemple de réalisation montré sur les figures, l'unité de commande de l'afficheur 11 est programmée pour que les positions 30 d'affichage successives de la cible 20 soient alignées sur cinq lignes.

Alternativement, les positions de la cible peuvent être alignées selon au moins deux colonnes.

De manière générale, les positions de la cible 20 peuvent être alignées selon des lignes parallèles de direction quelconque, notamment sensiblement horizontale ou verticale pour l'individu 1.

De préférence encore, chaque ligne L1, L2, ou alternativement chaque colonne, comprend au moins trois positions alignées de ladite cible (cas des positions 35, 36, 37, 38, 39 pour la ligne L2 de la figure 3).

Afin que le protocole de suivi visuel soit le plus représentatif d'une lecture par le porteur, il est avantageusement prévu que le protocole de suivi visuel décrive un trajet de lecture qui soit conforme à celui défini par un système d'écriture donné, de manière à reproduire le déplacement du regard de l'individu pendant la lecture conformément au système d'écriture.

Le trajet de lecture peut être ici défini comme le chemin, au niveau de l'afficheur 11, balayé par la direction de regard de l'individu 1 lorsqu'il regarde la séquence des positions 30 prises par la cible 20 visuellement prépondérante.

Le schéma de lecture adopté par un individu est lié non seulement à la nature ou aux propriétés spécifiques du texte, mais aussi aux spécificités de chaque écriture.

On notera d'ailleurs que les différentes écritures peuvent être classifiées de façon fonctionnelle (écriture alphabétique, syllabique ou logographique) et directionnelle (sens horizontal et vertical d'écriture et/ou de lecture).

On prévoit donc dans le dispositif de test que l'unité de commande mémorise un sens de parcours vertical SV et horizontal SH (voir figure 3) privilégié du protocole de suivi visuel.

Ce sens de parcours vertical et horizontal privilégié est préalablement déterminé en fonction des caractéristiques de l'individu, et en particulier sa capacité à lire un texte selon un système d'écriture donné.

Par exemple, lorsque le dispositif de test est utilisé par un français qui lit de droite à gauche et de haut en bas, le sens de parcours horizontal mémorisé par l'unité de commande est un sens de parcours allant de la gauche de l'écran 11 à la droite de l'écran 11, et le sens de parcours vertical mémorisé par l'unité de commande est un sens de parcours allant du haut de l'écran 11 au bas de l'écran 11.

Aussi, dans un mode de réalisation préféré, les lignes L1, L2 sensiblement parallèles le long desquelles sont alignées les positions 35, 36, 37, 38, 39 de la cible 20 s'étendent sensiblement horizontalement, le sens de parcours du protocole de suivi visuel étant identique pour toutes les lignes prises successivement de la plus haute à la plus basse, de gauche à droite (ou de droite à gauche pour une écriture de droite à gauche comme l'arabe ou l'hébreu).

De la même manière, lorsque le dispositif de test est utilisé par un mongolien, qui lit de haut en bas et de droite à gauche, le sens de parcours vertical mémorisé par l'unité de commande est un sens de parcours allant du haut de l'écran 11 au bas de l'écran 11, et le sens de parcours horizontal mémorisé par l'unité de commande est un sens de parcours allant de la droite de l'écran 11 à la gauche de l'écran 11.

Aussi, dans un mode de réalisation adapté à ce système d'écriture, les lignes sensiblement parallèles le long desquelles sont alignées les positions prédéterminées de la cible s'étendent sensiblement verticalement, le sens de parcours du protocole de suivi visuel étant identique, de haut en bas ou de bas en haut, pour toutes les lignes prises successivement de droite à gauche.

De manière avantageuse, l'unité de commande du dispositif de test 10 est programmée pour permettre la sélection du protocole de suivi visuel parmi une pluralité de protocoles de suivi visuel enregistrée dans une base de données locale ou distante, dans laquelle un sens de parcours est enregistré en association avec le protocole de suivi visuel auquel il correspond.

Ainsi, l'individu en fonction de ses caractéristiques propres de lecture et/ou d'écriture peut choisir le protocole visuel qui lui correspond, de sorte qu'il est dans des conditions naturelles proches de la lecture lors de la réalisation du test visuel. On s'assure alors de mettre en place ses mécanismes et ses stratégies de lecture afin de récupérer la posture la plus représentative de l'utilisation de sa vision de près.

Afin de reproduire le schéma de lecture tel que décrit plus haut, avec fixations, saccades et rétro-saccades, il est prévu que l'unité de commande de l'afficheur 11 affiche la cible 20 selon un protocole de suivi visuel préférentiel.

Aussi, il est prévu que l'unité de commande impose, dans chaque position du protocole de suivi visuel, que la cible 20 s'affiche pendant une durée prédéterminée. On entend par là que la cible 20 est maintenue affichée (cas de la position 34 de la figure 3) fixement sur l'écran de manière à ce qu'on force l'individu 1 à fixer son regard sur la cible 20, ce qui correspond à une fixation dans le trajet de lecture de l'individu 1.

De manière avantageuse, la cible est fixe pendant la durée prédéterminée, c'est-à-dire que la position de la cible 20 pendant cette durée prédéterminée ne change pas, avant le passage à la position suivante du trajet de lecture.

De préférence, cette durée prédéterminée est comprise entre 50 millisecondes et 1 seconde, ce qui correspond typiquement à des temps de fixation standard.

La durée prédéterminée peut également varier au cours du trajet de lecture, ceci rendant compte du fait que la fixation du regard de l'individu 1 sur un mot lors d'une lecture réelle peut dépendre du mot (taille, longueur) et du niveau de compréhension (mot mal connu ou inconnu, mot ou caractère peu déchiffrable, mot mal orthographié, etc...).

De manière avantageuse également, il est prévu que l'unité de commande impose un délai prédéterminé entre les affichages de la cible 20 dans deux positions successives 31, 32 (voir figure 3) du protocole de suivi visuel.

De cette façon, on peut simuler grâce au dispositif de test 10 les saccades ou rétro-saccades existant lors du trajet de lecture de l'individu 1. Comme précédemment, on peut prévoir que l'unité de commande fasse varier le délai prédéterminé au cours du protocole de suivi visuel.

Ceci permet de rendre compte que la vitesse de lecture de l'individu 1 peut varier au cours de la lecture d'un texte.

Ceci permet également d'envisager les cas où la direction de regard de l'individu passe d'une ligne à une autre, comme c'est le cas par exemple de la position 51 à la position 52 de la figure 3, le retour à la ligne nécessitant plus de temps dans la mesure où la variation de direction de regard de l'individu est plus importante.

Il est alors possible de prévoir deux cas pour la cible lors du délai prédéterminé.

Dans un mode de réalisation, on peut prévoir que la cible est invisible pendant le délai prédéterminé. Ceci correspond au cas des positions 31 et 32 de la figure 3 où la cible 20 « saute » (le saut étant représenté par la flèche pointillée 40) de la position 31 à la position suivante 32. Ce mode de réalisation permet de rendre compte du regard de l'individu qui saute de mot en mot lors de la lecture d'un texte.

Dans un mode de réalisation alternatif, on peut prévoir que la cible est visible pendant le délai prédéterminé et se déplace entre les deux positions successives correspondantes du protocole de suivi visuel, de l'une à l'autre. Ceci correspond au cas des positions 53 et 54 où la cible se déplace (le déplacement étant représenté par la flèche en pointillés 49), tout en restant visible (cf. cibles 21, 22), de la position 53 correspondant à la première position de la quatrième ligne du protocole à la position 54 correspondant ici à la deuxième position de la quatrième ligne du protocole. Cet autre mode de réalisation, quant à lui, permet de rendre compte du regard de l'individu qui parcourt le mot dans son entier avant de passer au suivant lors de la lecture.

De manière avantageuse, le dispositif de test 10 de l'invention est tel que l'unité de commande impose que deux positions successives 35, 36, 37, 38, 39 du protocole de suivi visuel sont séparées d'une distance EM1, EM2, EM3, EM4 inférieure à 10 centimètres. De cette façon, lors du test visuel, on ne sollicite pas l'individu 1 de telle sorte que la variation de sa direction de regard ne soit pas trop importante, ce qui en condition de lecture est généralement le cas.

Préférentiellement, il est par ailleurs prévu que l'unité de commande impose que la distance EM1, EM2, EM3, EM4 séparant deux positions 35, 36, 37, 38, 39 successives du protocole de suivi visuel varie le long du protocole de suivi visuel. Ceci permet d'adapter l'écart entre les cibles 20 affichées en fonction de l'empan moyen des mots pour un système d'écriture donné.

Dans un autre mode de réalisation, l'unité de commande est programmée pour que l'affichage de la cible 20 dans deux positions successives du protocole de suivi visuel suive le sens de parcours privilégié, horizontal et/ou vertical, au moins six fois sur dix. Ceci est illustré sur la figure 3 sur laquelle on a représenté dans le protocole de suivi visuel, des sens de parcours, représentés par les flèches pointillées 43, 45, 48, qui vont non pas de gauche à droite comme le sens de parcours horizontal SH privilégié, mais de droite à gauche.

Il est ainsi possible grâce à cela de simuler les mouvements de rétro-saccades précédemment décrits lors de la lecture d'un texte par l'individu 1. En effet, ici quatre fois sur dix, le mouvement des yeux 3 de l'individu 1 suivant la cible 20 du regard entre deux positions successives se fait dans le sens opposé au sens privilégié de parcours.

Comme pour les mouvements de saccades détaillés ci-dessus, la cible 20 peut passer d'une position à la suivante, dans un sens de parcours opposé au sens de parcours privilégié, soit en sautant d'une position à l'autre (cible invisible), soit en se déplaçant de l'une à l'autre (cible visible).

On va maintenant décrire, en référence aux figures 4 à 18, une méthode de détermination d'au moins un paramètre de conception optique d'une lentille ophtalmique destinée à équiper une monture choisie par l'individu, en fonction du comportement visuel de celui-ci, cette méthode utilisant le dispositif de test décrit ci-dessus.

Selon l'invention, la méthode de détermination comprend les étapes suivantes :
a) on demande à l'individu d'effectuer une tâche visuelle dans laquelle il regarde la cible affichée par ledit afficheur du dispositif d'affichage, les positions de ladite cible étant prédéterminées dans un référentiel lié à un appareil de capture d'image,
b) on capture, au moyen dudit appareil de capture d'image, des images de la tête de l'individu regardant ladite cible, chaque image correspondant à une position prédéterminée de ladite cible,
c) on détermine, à partir d'une partie au moins des images de la tête de l'individu, des positions de la tête de l'individu dans un référentiel lié audit appareil de capture d'image ou des directions de regard de l'individu dans un référentiel lié à la tête de l'individu, chaque position de la tête ou direction de regard de l'individu étant associée à la position de ladite cible pour laquelle l'image correspondante de la tête de l'individu a été capturée,
d) on déduit ledit paramètre de conception optique recherché à partir desdites positions de tête ou direction de regard de l'individu.

La figure 4 représente un diagramme logique de différentes étapes pouvant être mises en œuvre de manière avantageuse lors de la méthode de détermination de l'invention. Ces différentes étapes peuvent être regroupées selon six blocs 100, 110, 120, 130, 140, et 150 comprenant un ou plusieurs sous-blocs représentant chacun une étape particulière.

En pratique, la tablette 10, ou un ordinateur local ou distant, est programmée pour accomplir les étapes décrites ci-dessous.

### Bloc 100

Dans une étape d'identification préalable, représentée par le bloc 100 de la figure 4, on demande à l'individu de renseigner son nom et son prénom. Cette étape peut être réalisée au moyen de la tablette même, par exemple en affichant sur l'écran 11 de la tablette 10 des rubriques à compléter par l'individu 10.

Lors de cette étape d'identification, on demande également à l'individu de sélectionner en fonction de ses préférences ou caractéristiques de lecture, un système d'écriture initial en précisant les sens de parcours horizontal et vertical qu'il faut privilégier : de gauche à droite ou de droite à gauche, et de haut en bas ou de bas en haut.

Enfin, on mémorise une vitesse de lecture de l'individu. Cette vitesse de lecture peut être évaluée en demandant à l'individu d'indiquer, par exemple en cliquant sur une icône affichée sur l'écran, si sa vitesse de lecture est lente, normale, ou bien rapide. En fonction de cette vitesse de lecture, la durée prédéterminée d'affichage de la cible pourra être ajustée (voir blocs 113 et 118 ci-après).

En variante, cette vitesse de lecture pourrait être évaluée à partir de la lecture d'un texte réel, de longueur suffisante afin que cette vitesse de lecture corresponde à la vitesse de lecture moyenne sur l'ensemble du texte lu. Avantageusement, le texte lu peut être utilisé pour déterminer les positions d'affichage de la cible visuellement prépondérante du protocole de suivi visuel (cf. ci-dessus).

On peut également envisager de faire un entraînement avec une vitesse moyenne, demander à l'individu si cette vitesse moyenne était trop ou pas assez rapide et ajuster la vitesse en fonction de sa réponse.

L'ensemble de ces données personnelles peuvent également être automatiquement récupérées d'une base de données à partir de la transmission au serveur de base de données du nom et prénom de l'individu et éventuellement d'autres données personnelles telles que âge, adresse, etc...

### Bloc 110

Les étapes regroupées dans le bloc 110 de la figure 4 visent à construire un référentiel lié à la tête 4 de l'individu 1, dit *« référentiel posture VL »* ou *« repère CRO* », dans lequel la tête 4 de l'individu 1 présente une position et une orientation fixe et auquel on associe un repère, de préférence orthonormé, ayant une origine et trois axes non liés.

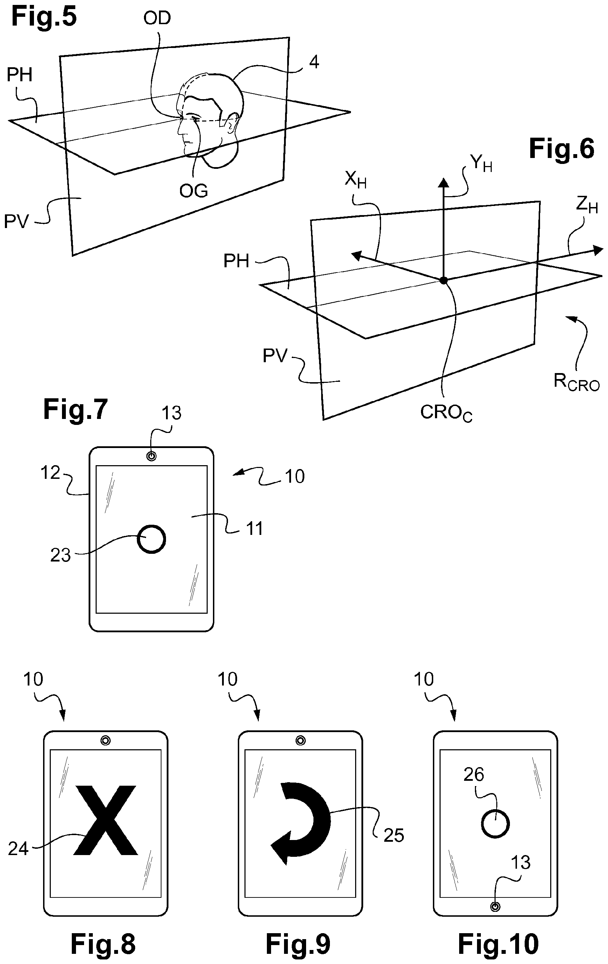

Les figures 5 et 6 illustrent comment est construit ce repère CRO.

En particulier, on a représenté sur la figure 5 un plan vertical PV correspondant à un plan sagittal de la tête 4 de l'individu 1 qui est le plan vertical passant par une médiatrice des deux yeux droit et gauche OD, OG de l'individu 1.

Cette médiatrice des yeux OD, OG est un axe qui passe au milieu d'un segment qui est défini par le centre de rotation de l'œil droit OD (ci-après référencé CROD) et le centre de rotation de l'œil gauche OG (ci-après référencé CROG) et qui est parallèle au plan de Francfort de la tête 4 de l'individu 1.

Le plan de Francfort de la tête de l'individu est défini comme le plan passant par les points orbitaires inférieurs de l'individu 1 et le porion de l'individu 1, le porion étant le point du crâne le plus élevé du conduit auditif, qui correspond au tragion de l'oreille. Pour la détermination du plan de Francfort, on considère que l'individu est dans une position orthostatique, dans laquelle il réalise un minimum d'efforts. Cette position correspond à une posture naturelle, ci-après désignée *« posture VL ».*

Dans cette position naturelle, la direction de regard de l'individu est alors la direction de regard primaire, c'est-à-dire qu'il regarde droit devant lui. Le plan de Francfort est alors généralement horizontal.

On définit par ailleurs un plan horizontal PH qui est, d'une part, parallèle au plan de Francfort de la tête 4 de l'individu 1 et, d'autre part, contient les centres de rotation CROD, CROG des yeux OD, OG de l'individu 1.

À partir de la posture VL de l'individu 1, c'est-à-dire de la connaissance de l'orientation du plan de Francfort, et des centres de rotation CROD, CROG des yeux OD, OG de l'individu 1, il est possible de construire le repère CRO lié à la tête 4 de l'individu 1, ci-après référencé R_{CRO}, en choisissant :
- une origine qui est l'un des centres de rotation CROD, CROG de l'œil droit OD ou de l'œil gauche OG de l'individu 1 ou un barycentre de ces centres de rotation CROD, CROG ;
- un premier axe qui passe par l'origine et les centres de rotation droit et gauche CROD, CROG,
- un deuxième axe qui passe par l'origine et qui est parallèle à la direction de regard primaire de l'individu 1, et
- un troisième axe qui passe par l'origine et qui est perpendiculaire aux premier et deuxième axe (le troisième axe correspond au « produit vectoriel » du deuxième axe avec le premier axe).

Dans le mode de réalisation particulier détaillé ici, l'origine du repère R_{CRO} est choisie comme étant le point situé au milieu du segment joignant le centre de rotation CROD de l'œil droit OD et le centre de rotation CROG de l'œil gauche OG de l'individu 1. En d'autres termes, ce point d'origine, désigné ci-après *« CRO cyclope* » et référencé CROc, correspond à l'isobarycentre des centres de rotation CROD, CROG des yeux OD, OG de l'individu 1.

Les trois axes X_{H}, Y_{H}, Z_{H}, du repère R_{CRO} sont également représentés sur la figure 6.

L'axe X_{H} (premier axe) est ici orienté du centre de rotation gauche CROG vers le centre de rotation droit CROD. L'axe X_{H} est donc contenu dans le plan horizontal PH parallèle au plan de Francfort. Une orientation opposée est également possible.

L'axe Z_{H} (deuxième axe) est situé dans le plan vertical PV de la tête 4 de l'individu 1 et est parallèle au plan de Francfort. Il est donc parallèle à la direction de regard primaire lorsque l'individu 1 est dans une position naturelle, c'est-à-dire en posture VL. L'axe Z_{H} s'étend ici dans une direction s'éloignant de la tête 4 de l'individu 1 (vers l'arrière).

L'axe Y_{H} (troisième axe) s'étend, quant à lui, dans le plan sagittal vertical PV de la tête 4 de l'individu 1 et est perpendiculaire au plan de Francfort. L'axe Y_{H} est donc bien perpendiculaire à l'axe X_{H} et à l'axe Z_{H}. Il est ici orienté vers le haut, de sorte que le repère R_{CRO} est direct.

On notera que le repère R_{CRO} lié à la tête 4 de l'individu 1 et que donc ce repère R_{CRO} bouge avec la tête 4 de l'individu 1, la position et l'orientation de ce repère R_{CRO} changeant par rapport à un repère absolu ou de référence qui ne serait pas lié à la tête 4 de l'individu 1 en fonction des mouvements de la tête 4 de l'individu 1.

Comme mentionné plus haut, le bloc 110 est destiné à construire le repère R_{CRO} à partir des données fournies par l'individu 1 ou bien à partir de mesures réalisées sur l'individu 1.

Ainsi, dans une étape représentée par le sous-bloc 111, on vérifie si pour l'individu 1 identifié précédemment (voir bloc 100), les positions des centres de rotation CROD, CROG et la posture VL sont disponibles.

Si ces données sont disponibles, alors on passe à une étape de création du référentiel de posture VL telle que décrite ci-dessus et représentée par le sous-bloc 112. Les données peuvent être disponibles localement, c'est-à-dire enregistrées dans une mémoire de la tablette 10, ou bien disponibles à distance, c'est-à-dire enregistrées dans une base de données sur laquelle des requêtes peuvent être effectuées pour récupérer lesdites données.

Si ces données ne sont pas disponibles, alors il est nécessaire d'une part de déterminer les positions respectives des centres de rotation CROD, CROG (sous-bloc 113) des yeux OD, OG de l'individu 1, et d'autre part de déterminer la posture VL (sous-bloc 114) de l'individu 1.

La détermination des positions des centres de rotation CROD, CROG peut être réalisée selon le principe connu en soi et exposé par exemple dans le document FR 2914173, dont un équivalent en anglais est le document US 2010/0128220.

Lors de l'étape de détermination des centres de rotation CROD, CROG (sous-bloc 113), l'individu 1 porte, sur sa tête 4, solidaire de la tête 4, un système de repérage (référentiel métrologique) ou « *clip »* qui comprend des éléments de repérage (marqueurs) détectables lors d'une capture d'image de la tête 4 de l'individu 1.

En résumé, on capture au moins deux images de la tête 4 de l'individu 1 au moyen d'un appareil de capture d'images :
- une première image lorsque l'individu regarde l'appareil de capture d'images en se positionnant de face, en regardant au loin droit devant (posture VL), et
- une deuxième image lorsque l'individu regarde l'appareil de capture d'images en se positionnant de trois-quart.

Si la tablette 10 est munie d'une caméra arrière, celle-ci peut avantageusement servir d'appareil de capture d'images. Sinon, on peut prévoir un appareil de capture d'images indépendant de la tablette.

À partir d'un traitement des deux images capturées (voir document FR 2914173), on déduit les positions des centres de rotation CROD, CROG dans un référentiel lié au système de repérage.

Il est alors possible de déterminer un point particulier, ci-après désigné centre de rotation « cyclope » ou « CRO cyclope » et référencé CRO_{C}, qui est l'isobarycentre des deux centres de rotation CROD, CROG précédemment déterminés.

Dans l'étape suivante de détermination de la posture VL, représentée par le sous-bloc 114, on réutilise les positions des centres de rotation CROD, CROG ainsi que la première image capturée de face pour déterminer la posture VL de l'individu 1. On peut également prévoir de compenser l'inclinaison de la tablette 10 lors de cette dernière détermination (voir par exemple documents US 8231220 et US 7950800).

Ainsi, à l'issue des étapes représentées par les sous-blocs 113 et 114, les données utiles pour la création du référentiel posture VL sont disponibles et l'étape de création décrite ci-dessus en référence au sous-bloc 112 peut être mise en œuvre.

### Bloc 120

Ensuite, dans un ensemble d'étapes représenté par le bloc 120, on effectue de préférence des réglages de la tablette 10 permettant d'améliorer la précision et la fiabilité des mesures de la position de la tête 4 de l'individu 1 ou des directions de regard de l'individu 1.

Ainsi, dans une étape représentée par le sous-bloc 121 et illustrée sur la figure 7, on détermine les positions des marqueurs du clip dans le champ de la caméra frontale 13 de la tablette 10, lorsque la caméra frontale 13 est orientée en haut.

On affiche à cet effet au centre de l'écran 11 un élément, ici un rond 23, et on demande à l'individu 1, qui tient en main la tablette 10, de regarder le rond 23.

En variante, on pourrait afficher cet élément à la première position d'affichage (voir position 31 de la figure 3) de la cible.

Dans une étape suivante (sous-bloc 122), on commande la caméra frontale 13 de la tablette 10 pour acquérir des images de la tête 4 de l'individu 1 équipé du système de repérage précédemment utilisé et on détermine, à partir d'un traitement des images acquises si :
i) la position des marqueurs du clip est depuis un temps prédéfini dans les deux tiers supérieurs du champ de la caméra 13 ;
ii) la position des marqueurs du clip est depuis un temps défini dans le tiers inférieur du champ de la caméra 13 ; ou
iii) la position des marqueurs du clip est introuvable au delà d'un temps limite.

Dans le cas i), on peut alors poursuivre vers l'étape représentée par le sous-bloc 131 de la phase d'entraînement représentée par le bloc 130.

Dans le cas iii) ci-dessus, on poursuit vers l'étape représentée par le sous-bloc 123 lors de laquelle on affiche sur l'écran 11 une figure, ici une croix 24 (voir figure 8), indiquant à l'individu 1 qu'une erreur de mesure a été détectée pour le champ de la caméra 13.

Toujours dans le cas iii) ou bien dans le cas ii), on affiche sur l'écran 11 une figure, ici une flèche 25 en forme d'arc de cercle (voir figure 9), indiquant à l'individu 1 de retourner la tablette 10 de façon à positionner la caméra frontale 13 vers le bas.

De préférence, il est prévu lors de cette étape de vérifier que la tablette 10 a bien été retournée par l'individu 1 (voir figure 10). Avantageusement, cette vérification peut être effectuée grâce à des capteurs d'orientation de la tablette 10, par exemple du type gyromètre.

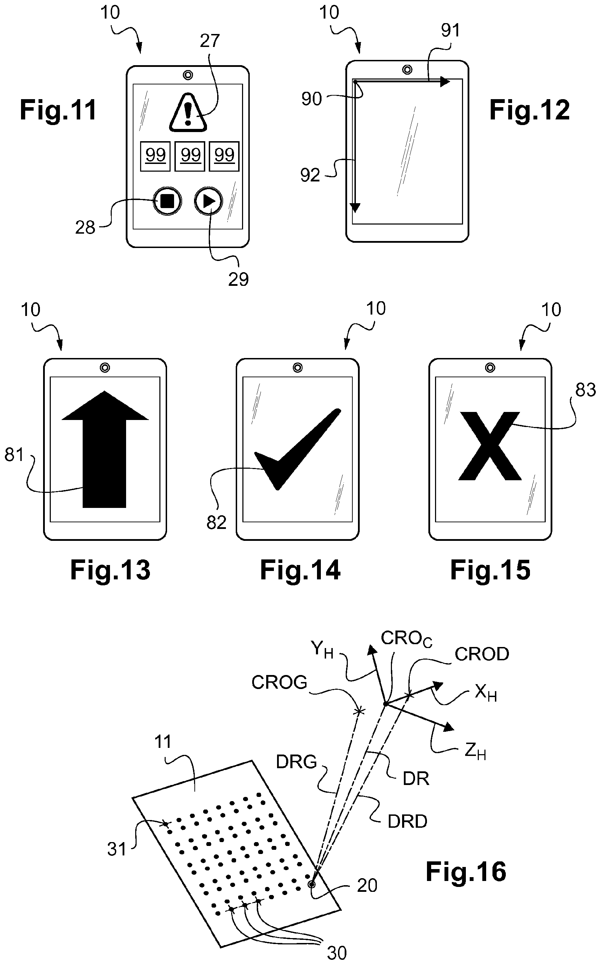

Une fois la vérification faite, on passe à l'étape représentée par le sous-bloc 124 et illustrée sur la figure 11, on détermine les positions des marqueurs du clip dans le champ de la caméra frontale 13 de la tablette 10, lorsque la caméra frontale 13 est orientée en bas.

On affiche alors au centre de l'écran 11 un élément, ici un rond 26, et on demande à l'individu 1, qui tient en main la tablette 10, de regarder ce rond 26.

En variante, on pourrait afficher cet élément à la première position d'affichage (voir position 31 de la figure 3) de la cible.

Dans une étape suivante (sous-bloc 125), on commande la caméra frontale 13 de la tablette 10 pour acquérir des images de la tête 4 de l'individu 1 équipé du système de repérage précédemment utilisé et tenant la tablette entre ses mains 2.

On détermine alors, à partir d'un traitement des images acquises si :
i) la position des marqueurs du clip est depuis un temps prédéfini dans le champ de la caméra 13 ;
ii) la position des marqueurs du clip est introuvable au delà d'un temps limite.

Dans le cas i), on peut alors poursuivre vers l'étape représentée par le sous-bloc 131 de la phase d'entraînement représentée par le bloc 130.

Dans le cas ii) ci-dessus, on poursuit vers l'étape représentée par le sous-bloc 126 lors de laquelle on affiche sur l'écran 11 un symbole 27 (voir figure 11), signalant à l'individu 1 une non-détection du clip ou une erreur de calcul.

La tablette 10 affiche alors un message et demande de recommencer l'étape en cours (voir bouton 29) ou de mettre fin au test (voir bouton 28).

De plus, des pictogrammes (non représentés mais qui seraient situés dans les cases vierges 99 de la figure 11) rappelant l'essentiel des bonnes pratiques sont affichés.

Par exemple, ces pictogrammes peuvent rappeler à l'individu 1 de :
- faire attention à la présence de lumières gênantes qui peuvent être source d'éblouissement (par ex. plafonniers, grandes baies vitrées dans le dos de l'individu) ;
- dégager le champ de vision de la caméra frontale 13 qui pourrait être obstrué par le doigt d'une main ou bien un vêtement qu'il porte.

À l'issue des étapes du bloc 120 (phase de réglage), la caméra frontale 13 de la tablette 10 a été placée dans les meilleures conditions pour faire une mesure sans que l'individu 1 ne réajuste sa posture ou la tablette 10 lors du protocole de suivi visuel.

### Bloc 130

De préférence, les étapes 131, 132, 133, 134, 135 du bloc 130, qui correspond à une phase d'entraînement, sont réalisées avant la phase de mesure à proprement parler (étapes du bloc 140, voir figure 4).

Dans une première étape de la phase d'entraînement, soit on charge dans une mémoire prévue à cet effet dans l'unité de commande, soit on calcule un protocole de suivi visuel d'entraînement tiré au hasard parmi un ensemble de protocoles de suivi visuel accessibles.

Cet ensemble de protocoles de suivi visuel accessibles est déterminé en fonction des sens de lecture privilégiés renseignés par l'individu lors de l'étape d'initialisation (bloc 100, voir ci-dessus). Cet ensemble peut être disponible directement dans une mémoire de la tablette 10 ou bien téléchargé depuis un serveur distant.

Le protocole de suivi visuel comporte des données représentatives de la position de la cible 20 visuellement prépondérante à afficher sur l'écran 11 de la tablette 10.

Ces données représentatives comprennent :
- un index (entier naturel) de la position de la cible 20 ;
- un repère temporel d'affichage de la cible 20 repéré et exprimé en secondes par rapport au début du protocole de suivi visuel ;
- deux coordonnées des positions d'affichage de la cible 20 sur l'écran 11, ces coordonnées étant exprimées par rapport à un repère d'écran, ci-après référencé R_{SCR}, ayant pour origine le coin supérieur gauche 90 de l'écran 11 (voir figure 12) et deux axes 91, 92 perpendiculaires entre eux et dirigés selon les colonnes et les lignes de l'écran 11.

En d'autres termes, à chaque instant, la position de la cible 20 est connue dans le repère d'écran R_{SCR} lié à l'appareil de capture d'image 13.

Une fois le protocole de suivi visuel chargé, on affiche tout d'abord sur l'écran 11 de la tablette 10 la cible 20 dans la première position (index = 0) du protocole de suivi visuel pendant un temps prédéfini (sous-bloc 132). On peut prévoir qu'un indicateur sonore ou une vibration de la tablette 10 indique le début du protocole de suivi visuel.

Ensuite (sous-bloc 133), on affiche la cible 20 visuellement prépondérante dans ses positions successives en fonction du protocole de suivi visuel d'entraînement choisi. La vitesse de parcours du protocole peut être ajustée en fonction des informations recueillies lors de la phase d'initialisation (bloc 100) concernant la vitesse de lecture de l'individu 1.

Lors de cette étape, l'utilisateur 1 s'habitue à suivre du regard la cible 20 visuellement prépondérante prenant séquentiellement l'ensemble des positions d'affichage prédéterminées pour le protocole de suivi visuel d'entraînement chargé.

Comme expliqué précédemment, les positions d'affichage de la cible 20 sont adaptées pour simuler la lecture d'un texte par l'individu 1. Ainsi, lors de la phase d'entraînement (bloc 130), l'utilisateur 1 va progressivement adopter une posture correspondant à sa posture naturelle en situation de pseudo-lecture.

Lorsque l'étape précédente (sous-bloc 133) est terminée, on peut de préférence afficher sur l'écran 11 (voir figure 13, sous-bloc 134) une figure indiquant la fin du cycle. Un indicateur sonore ou une vibration peut également indiquer la fin du cycle.

À cette étape (sous-bloc 134), la figure affichée, ici une flèche 81 dirigée vers le haut, représente un indicateur incitant l'individu à regarder au loin.

Cette figure 81 reste affichée sur l'écran 11 pendant un temps défini avant de disparaître. La tablette 10 n'affiche alors rien sur l'écran 11 et attend un temps défini.

Après cette attente, on vérifie (sous-bloc 135) l'état d'avancement de la phase d'apprentissage. En particulier, si l'utilisateur n'a réalisé qu'une fois le protocole de suivi visuel, on recommence de préférence les étapes 133 et 134 une seconde fois. Sinon, on passe à la première étape (sous-bloc 141) de la phase de mesure proprement dite (bloc 140).

### Bloc 140

Lors de cette première étape 141, qui est similaire à la première étape 131 de la phase d'entraînement (bloc 130), soit on charge dans une mémoire prévue à cet effet dans l'unité de commande, soit on calcule un protocole de suivi visuel de mesure tiré au hasard parmi l'ensemble des protocoles de suivi visuel accessibles précédemment déterminé.

Ce protocole de suivi visuel comporte des données similaires qui sont représentatives de la position d'affichage de la cible 20 visuellement prépondérante sur l'écran 11 de la tablette 10 lors de la phase de mesure.

Ces données comportent notamment les positions d'affichage de la cible 20 visuellement prépondérante qui sont prédéterminées et connues dans le repère R_{SCR} lié à l'appareil de capture d'image (caméra frontale 13) du dispositif de test (tablette 10).

Dans une étape a) de la méthode de détermination selon l'invention représentée par les sous-blocs 142 et 143 de la figure 4, on demande à l'individu 1 d'effectuer une tâche visuelle dans laquelle il regarde la cible 20 affichée par l'écran 11 de la tablette 10 (voir figure 16). Les positions 30 d'affichage de cette cible 20 sont prédéterminées dans le repère R_{SCR} (cf. origine 90 et axes 91, 92 de la figure 3) lié à la caméra frontale 13.

Plus précisément, on affiche tout d'abord (sous-bloc 142), sur l'écran 11 de la tablette 10, la cible 20 dans la première position 31 (index = 0) du protocole de suivi visuel chargé pour la mesure pendant un temps prédéfini.

On peut également prévoir qu'un indicateur sonore ou une vibration de la tablette 10 indique le début du protocole de suivi visuel.

Ensuite (sous-bloc 143), on affiche la cible 20 visuellement prépondérante dans ses positions 30 successives en fonction du protocole de suivi visuel de mesure. La vitesse de parcours du protocole peut être ajustée en fonction des informations recueillies lors de la phase d'initialisation (bloc 100) concernant la vitesse de lecture de l'individu 1.

Lors de cette étape a), l'utilisateur suit du regard la cible 20 visuellement prépondérante prenant séquentiellement l'ensemble des positions 30 d'affichage prédéterminées pour le protocole de suivi visuel chargé (voir figure 16 où la cible 20 est affichée à la dernière position du protocole de suivi visuel).

Lors de l'étape b) de la méthode de détermination, représentée par le sous-bloc 144, on capture, au moyen de la caméra frontale 13 de la tablette 10 tournée vers la tête 4 de l'individu 1, des images de la tête 4 de l'individu 1 regardant la cible 20 qui se déplace sur l'écran 11 suivant le protocole de suivi visuel chargé.

Avantageusement, la caméra frontale 13 déclenche une capture d'image de la tête 4 de l'individu 1 avec un décalage de capture par rapport au moment où la cible 20 s'affiche aux positions 20 prédéterminées du protocole de suivi visuel sur l'écran 11. Ce décalage peut être nul, ou bien de préférence faible, par exemple inférieur à 200 millisecondes. Ceci permet de prendre en compte le temps de réaction et de déplacement des yeux 3 de l'individu 1 lors d'un changement de position 30 de la cible 20 sur l'écran 11.

Selon une variante, la caméra frontale peut également réaliser une séquence vidéo en continu, par exemple à une cadence de vingt images par seconde, et extraire de la séquence vidéo la meilleure image donnant la meilleure information sur le comportement visuel de l'individu lors de l'affichage de la cible à la position cible correspondante.

Chaque image capturée par la caméra frontale 13 de la tablette 10 correspond ainsi à une position prédéterminée de la cible 20 visuellement prépondérante, dont la position 30 dans le repère R_{SCR} lié à l'appareil de capture d'image 13 est parfaitement connue (voir sous-bloc 141 ci-dessus et figure 16).

Alors, dans l'étape c) de la méthode de détermination selon l'invention, on détermine, à partir d'une partie au moins des images de la tête 4 de l'individu 1, soit les positions de la tête 4 de l'individu 1 dans le repère R_{SCR} lié à la caméra frontale 13, soit les directions de regard de l'individu 1 dans le référentiel R_{CRO} lié à la tête 4 de l'individu 1, chaque position de la tête 4 ou direction de regard de l'individu 1 étant associée à la position 30 de la cible 20 pour laquelle l'image correspondante de la tête 4 de l'individu 1 a été capturée.

À cet effet, il est prévu des moyens de traitement d'images de la tablette 10, constitués par exemple par le processeur de la tablette 10, qui détecte dans les images capturées de la tête 4 de l'individu 1 les marqueurs du clip porté par l'individu 1 sur sa tête 4.

On détermine alors pour chaque image capturée, c'est-à-dire pour chaque position 30 de la cible 20 du protocole de suivi visuel, la position et l'orientation du clip dans le repère R_{SCR} lié à la caméra frontale 13 par exemple en utilisant le procédé décrit dans le document FR 2914173 (dont un équivalent en anglais est le document US 8360580).

Les positions des centres de rotation CROD, CROG des yeux de l'individu 1 par rapport au clip étant connues (voir sous-blocs 111 et 114), la position (coordonnées spatiales) et l'orientation (coordonnées angulaires) du repère R_{CRO} lié à la tête 4 de l'individu 1 sont également connues par rapport au clip.

Ceci est d'ailleurs illustré sur la figure 16 où l'on a représenté le repère R_{CRO} avec son origine au centre de rotation cyclope CRO_{C} (isobarycentre des centres de rotation CROD, CROG) et ses axes X_{H}, Y_{H}, Z_{H}.

Ainsi, par un changement de repère, il est possible de déterminer, pour chaque position 30 de la cible 20 du protocole de suivi visuel, la position et l'orientation de la tête 4 de l'individu 1 dans le repère R_{SCR} lié à la caméra frontale 13 de la tablette 10.

On peut également déterminer, pour chaque position 30 de la cible 20 du protocole de suivi visuel, les directions de regard DR de l'individu 1 dans le référentiel R_{CRO} lié à la tête 4 de l'individu 1, ces directions de regard DR joignant ici le centre de rotation cyclope CRO_{C}, origine du repère R_{CRO} lié à la tête 4 de l'individu 1, à la cible 20.

On peut également déterminer les directions de regard DRD, DRG (voir figure 16) relatives respectivement au centre de rotation droit CROD et au centre de rotation gauche CROG.

Dans un mode de réalisation préférentiel, préalablement à l'étape d), on réexprime alors, à partir des positions et orientations de la tête 4 ou des directions de regard DR de l'individu 1, les positions 70 de la cible 20 dans le repère R_{CRO} lié à la tête 4 de l'individu 1.

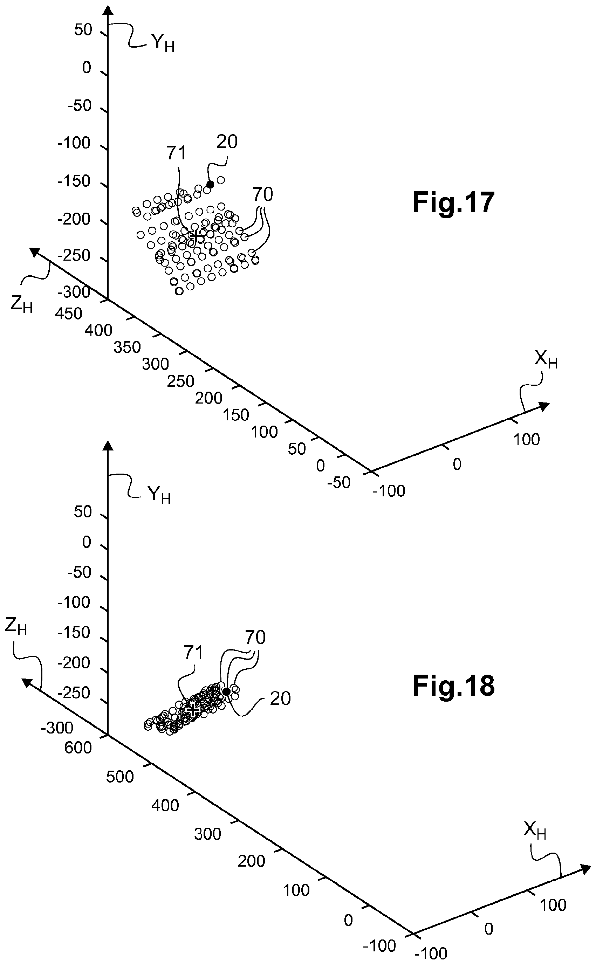

Ceci est illustré sur la figure 17 où l'on a représenté par rapport aux axes X_{H}, Y_{H}, Z_{H} du repère R_{CRO} lié à la tête 4 de l'individu 1 les positions 70 de la cible 20 dans ce repère R_{CRO}.

Compte tenu du fait non seulement que la position et l'orientation de la tête 4 de l'individu 1 change au cours du protocole de test visuel par rapport au repère R_{SCR} lié à l'appareil de capture d'image 13 mais aussi que l'individu 1 modifie la position et l'orientation de la tablette 10 au cours du test visuel, on comprend que les positions 70 relatives de la cible 20 dans le repère R_{CRO} lié à la tête 4 de l'individu 1 renseignent sur le comportement visuel de l'individu 1, en particulier sur sa propension à bouger plutôt sa tête 4 ou plutôt ses yeux 3 lors de la lecture d'un texte.

En effet, si l'individu 1 suit le protocole de suivi visuel modifiant beaucoup sa direction de regard DR, alors les positions 70 relatives de la cible 20 dans le repère R_{CRO} lié à la tête 4 de l'individu 1 sont semblables aux positions 30 relatives de la cible 20 dans le repère R_{SCR} lié à la caméra frontale 13. Ceci est le cas de la figure 17.

À l'inverse, si l'individu 1 suit le protocole de suivi visuel en conservant une direction de regard DR quasi fixe, alors les positions 70 de la cible 20 dans le repère R_{CRO} lié à la tête 4 de l'individu 1 sont regroupées. Ceci est le cas de la figure 18.

Par ailleurs, on peut associer à ces positions 70 de la cible 20 dans le repère R_{CRO} lié à la tête 4 de l'individu 1 un index et un repère temporel tel que décrit ci-dessus pour le sous-bloc 141.

Dans une étape d) de la méthode de détermination, représentée ici par le sous-bloc 146, on déduit le paramètre de conception optique recherché à partir des positions de tête 4 ou direction de regard DR de l'individu.

Dans le mode de réalisation particulier décrit, le paramètre de conception optique recherché est ici déduit à partir des positions 70 de la cible 20 dans le repère R_{CRO} lié à la tête 4 de l'individu 1.

Ce paramètre de conception optique peut comprendre par exemple la donnée des coordonnées du barycentre 71 des positions 70 de la cible 20 dans le repère R_{CRO} lié à la tête 4 de l'individu 1. Ce barycentre 71 définit une direction de regard moyenne de l'individu 1 joignant le centre de rotation cyclope CRO_{C} et ledit barycentre 71.

Cette direction de regard moyenne est représentative du comportement visuel de l'individu 1 en vision de près et en situation de lecture.

La donnée des coordonnées du barycentre 71 dans le repère R_{CRO} lié à la tête 4 de l'individu 1 peut donc être utilisée pour concevoir une lentille ophtalmique, notamment une lentille ophtalmique progressive, destinée à équiper une monture de lunettes choisie par l'individu 1.

Comme cela est décrit dans le document FR 3012952, il est par exemple possible, à partir du barycentre 71 et de la direction de regard moyenne, de positionner précisément un point de référence en vision de près (point de centrage VP) sur une lentille ophtalmique et également d'optimiser le design des surfaces avant et arrière de la lentille ophtalmique pour une meilleure adaptation de celle-ci à l'individu 1.

Grâce aux positions 70 de la cible 20 dans le repère R_{CRO} lié à la tête 4 de l'individu 1, on peut également déduire un paramètre de conception optique rendant compte de la tendance de l'individu 1 à bouger la tête 4 et/ou les yeux 3 lors d'une tâche de lecture.

Comme mentionné plus haut en référence aux figures 19 et 20, ce paramètre de conception optique peut correspondre à une donnée représentative de la dispersion des positions 70 de la cible 20 dans le référentiel R_{CRO} lié à la tête 4 de l'individu 1.

On peut déterminer cette dispersion en calculant, pour chaque position 70, la distance entre cette position 70 et la position du barycentre 71 précédemment défini.

Les positions 30 de la cible 20 s'étendant ici horizontalement et verticalement, on peut également déterminer un coefficient de dispersion horizontal et un coefficient de dispersion vertical. Le coefficient de dispersion horizontal quantifie les mouvements des yeux 3 de l'individu 1 de gauche à droite (ou de droite à gauche) lors du protocole de suivi visuel. Le coefficient de dispersion vertical quantifie quant à lui les mouvements des yeux 3 de l'individu 1 de haut en bas (ou de bas en haut) lors du protocole de suivi visuel.

D'autres paramètres de conception optique représentatifs du comportement visuel de l'individu peuvent bien évidemment être déterminés.

Dans une étape suivante, représentée ici par le sous-bloc 147, on vérifie que la détermination du paramètre de conception optique a été réalisée avec succès.

Par exemple, lorsque ce paramètre de conception optique comprend la donnée des coordonnées du barycentre 71 (voir figure 17) des positions 70 de la cible dans le repère lié à la tête 4 de l'individu 1, on peut vérifier que les coordonnées de ce barycentre 71 sont comprises dans des gammes spécifiques qui sont fonction des positions 30 de la cible 20 dans le repère R_{SCR} de la tablette et des amplitudes minimales et/ou maximales de mouvement de la tête 4 de l'individu 1.

Toute valeur déterminée en dehors de ces limites engendre une erreur. Deux cas sont alors possibles :
- si c'est la première fois que la mesure est faite alors l'erreur est maintenue et un signal d'erreur (ici une croix 83, voir figure 15) est affiché sur l'écran 11 de la tablette 10 (sous-bloc 148, voir aussi sous-bloc 126) ;
- si c'est la deuxième mesure et si une erreur est signalée alors l'erreur est annulée et toutes les valeurs sont figées à la limite minimale. Ce cas sera détecté comme une valeur aberrante et sera géré en conséquence.

Dans le cas où la vérification précédente se passe bien, on affiche sur l'écran un symbole de réussite (voir symbole 82 en forme de V sur la figure 14) et on passe à l'étape suivante qui est représentée par le sous-bloc 149 et qui consiste à encoder les différents paramètres de conception optique déduits dans un format déterminé.

On peut par exemple encoder chacune des coordonnées du barycentre 71 des positions 70 de la cible 20 dans le repère R_{CRO} lié à la tête 4 de l'individu 1 dans une base hexadécimale, sur un ou plusieurs octets, de préférence deux octets.

### Bloc 150

Lors d'une phase de transfert (bloc 150), les paramètres de conception optique sont transmis à un calculateur local ou distant destiné à utiliser lesdits paramètres en vue de la conception optique d'une lentille ophtalmique destinée à l'individu 1 et particulièrement bien adaptée à son comportement visuel, ici son comportement de lecture en vision de près.

## Revendications

1. Dispositif de test (10) du comportement visuel d'un individu (1) comportant :
- un afficheur (11) actif adapté à afficher au moins une cible (20) visuellement prépondérante en une pluralité de positions (30) variables au cours du temps et alignées selon au moins une ligne (L1, L2) ou une colonne, et
- une unité de commande de l'afficheur (11), programmée pour que les positions (30) d'affichage successives de la cible (20) suivent, au cours du temps, un protocole de suivi visuel, **caractérisé en ce que**:
toutes les positions (30) d'affichage successives sont telles que la cible (20) est dans le champs visuel de l'individu et,
a cible (20) visuellement prépondérante étant celle qui est adaptée à accrocher le regard dudit individu (1) suivant ladite cible (20) au cours du test visuel, ledit protocole de suivi visuel correspondant à la succession, dans le temps, des positions prises par la cible (20) visuellement prépondérante, ladite cible (20) imposant un protocole de suivi visuel à l'individu (1) qui regarde successivement dans une pluralité de directions particulières désirées qui sont chacune associées à une position (30) d'affichage particulière prise par la cible (20).

2. Dispositif de test (10) selon la revendication 1, dans lequel lesdites positions (30) de la cible (20) sont alignées selon au moins deux lignes (L1, L2) ou deux colonnes sensiblement parallèles.

3. Dispositif de test (10) selon l'une des revendications 1 à 2, dans lequel ladite pluralité comprend, sur chaque ligne ou colonne, au moins trois positions (35, 36, 37) alignées de ladite cible (20).

4. Dispositif de test (10) selon l'une des revendications 1 à 3, dans lequel l'unité de commande impose, dans chaque position (30) dudit protocole de suivi visuel, que la cible (20) s'affiche pendant une durée prédéterminée.

5. Dispositif de test (10) selon la revendication 4, dans lequel ladite durée prédéterminée est comprise entre 50 millisecondes et 1 seconde.

6. Dispositif de test (10) selon la revendication 4 ou 5, dans lequel ladite cible (20) est fixe pendant ladite durée prédéterminée.

7. Dispositif de test (10) selon l'une des revendications 1 à 6, dans lequel l'unité de commande impose un délai prédéterminé entre les affichages de la cible (20) dans deux positions (30) successives du protocole de suivi visuel.

8. Dispositif de test (10) selon la revendication 7, dans lequel ledit délai prédéterminé varie au cours du protocole de suivi visuel.

9. Dispositif de test (10) selon l'une des revendications 1 à 8, dans lequel ladite unité de commande impose que deux positions (30) successives du protocole de suivi visuel sont séparées d'une distance (EM1, EM2, EM3, EM4) inférieure à 10 centimètres.

10. Dispositif de test (10) selon l'une des revendications 1 à 9, dans lequel ladite unité de commande impose que deux positions (30) successives du protocole de suivi visuel sont séparées d'une distance (EM1, EM2, EM3, EM4) variant le long du protocole de suivi visuel.

11. Dispositif de test (10) selon l'une des revendications 1 à 10 dans lequel ladite unité de commande mémorise un sens de parcours vertical (SV) et horizontal (SH) privilégié du protocole de suivi visuel.

12. Dispositif de test (10) selon l'une des revendications 1 à 11, dans lequel le protocole de suivi visuel suit un trajet de lecture conforme à celui défini par un système d'écriture donné, de manière à reproduire le déplacement du regard de l'individu (1) pendant la lecture conformément au système d'écriture.

13. Méthode de détermination d'au moins un paramètre de conception optique d'une lentille ophtalmique destinée à équiper une monture choisie par l'individu (1), en fonction du comportement visuel de celui-ci, ladite méthode utilisant le dispositif de test (10) selon l'une des revendications 1 à 12 et comprenant les étapes suivantes :
a) on demande à l'individu (1) d'effectuer une tâche visuelle dans laquelle il regarde la cible (20) visuellement prépondérante affichée par ledit afficheur (11) du dispositif d'affichage et adaptée à accrocher le regard dudit individu (1) au cours du test visuel, ladite cible (20) imposant un protocole de suivi visuel à l'individu (1) qui regarde successivement dans une pluralité de directions particulières désirées qui sont chacune associées à une position (30) d'affichage particulière prise par la cible (20), ledit protocole de suivi visuel correspondant à la succession, dans le temps, des positions prises par la cible (20) visuellement prépondérante, les positions (30) de ladite cible (20) étant prédéterminées dans un référentiel lié à un appareil de capture d'image,
b) on capture, au moyen dudit appareil de capture d'image, des images de la tête de l'individu (1) regardant ladite cible (20), chaque image correspondant à une position (30) prédéterminée de ladite cible (20),
c) on détermine, à partir d'une partie au moins des images de la tête de l'individu (1), des positions de la tête de l'individu (1) dans un référentiel lié audit appareil de capture d'image ou des directions de regard (DR) de l'individu (1) dans un référentiel lié à la tête de l'individu (1), chaque position de la tête ou direction de regard de l'individu (1) étant associée à la position (30) de ladite cible (20) pour laquelle l'image correspondante de la tête de l'individu (1) a été capturée,
d) on déduit ledit paramètre de conception optique recherché à partir desdites positions de tête ou direction de regard de l'individu (1).

14. Méthode de détermination selon la revendication ' 13, selon laquelle, préalablement à l'étape d), on réexprime, à partir desdites positions déterminées de la tête ou des directions de regard de l'individu (1), les positions (70) de ladite cible (20) dans un référentiel lié à la tête de l'individu (1), et à l'étape d), on déduit ledit paramètre de conception optique recherché à partir desdites positions (70) de la cible (20) dans le référentiel lié à la tête de l'individu (1).

## Patentansprüche

1. Testvorrichtung (10) des Sehverhaltens einer Person (1), die aufweist:
- ein aktives Display (11), das geeignet ist, mindestens ein visuell ausschlaggebendes Ziel (20) in einer Vielzahl von Positionen (30) anzuzeigen, die im Laufe der Zeit variabel und gemäß mindestens einer Zeile (L1, L2) oder einer Spalte ausgerichtet sind, und
- eine Steuereinheit des Displays (11), die programmiert ist, damit die aufeinanderfolgenden Anzeigepositionen (30) des Ziels (20) im Laufe der Zeit einem visuellen Verfolgungsprotokoll folgen,
**dadurch gekennzeichnet, dass**:
alle aufeinanderfolgenden Anzeigepositionen (30) so sind, dass das Ziel (20) sich im Blickfeld der Person befindet, und
wobei das visuell ausschlaggebende Ziel (20) dasjenige ist, das geeignet ist, den Blick der Person (1), der während des visuellen Tests dem Ziel (20) folgt, auf sich zu ziehen, wobei das visuelle Verfolgungsprotokoll der zeitlichen Aufeinanderfolge der vom visuell ausschlaggebenden Ziel (20) eingenommenen Positionen entspricht, wobei das Ziel (20) der Person (1), die nacheinander in eine Vielzahl von gewünschten besonderen Richtungen blickt, die je einer vom Ziel (20) eingenommenen besonderen Anzeigeposition (30) zugeordnet sind, ein visuelles Verfolgungsprotokoll vorgibt.

2. Testvorrichtung (10) nach Anspruch 1, wobei die Positionen (30) des Ziels (20) gemäß zwei mindestens im Wesentlichen parallelen Zeilen (L1, L2) oder Spalten ausgerichtet sind.

3. Testvorrichtung (10) nach einem der Ansprüche 1 bis 2, wobei die Vielzahl auf jeder Zeile oder Spalte mindestens drei ausgerichtete Positionen (35, 36, 37) des Ziels (20) enthält.

4. Testvorrichtung (10) nach einem der Ansprüche 1 bis 3, wobei die Steuereinheit in jeder Position (30) des visuellen Verfolgungsprotokolls vorgibt, dass das Ziel (20) während einer vorbestimmten Dauer angezeigt wird.

5. Testvorrichtung (10) nach Anspruch 4, wobei die vorbestimmte Dauer zwischen 50 Millisekunden und 1 Sekunde liegt.

6. Testvorrichtung (10) nach Anspruch 4 oder 5, wobei das Ziel (20) während der vorbestimmten Dauer ortsfest ist.

7. Testvorrichtung (10) nach einem der Ansprüche 1 bis 6, wobei die Steuereinheit eine vorbestimmte Verzögerung zwischen den Anzeigen des Ziels (20) in zwei aufeinanderfolgenden Positionen (30) des visuellen Verfolgungsprotokolls vorgibt.

8. Testvorrichtung (10) nach Anspruch 7, wobei die vorbestimmte Verzögerung während des visuellen Verfolgungsprotokolls variiert.

9. Testvorrichtung (10) nach einem der Ansprüche 1 bis 8, wobei die Steuereinheit vorgibt, dass zwei aufeinanderfolgende Positionen (30) des visuellen Verfolgungsprotokolls um einen Abstand (EM1, EM2, EM3, EM4) kleiner als 10 Zentimeter getrennt sind.

10. Testvorrichtung (10) nach einem der Ansprüche 1 bis 9, wobei die Steuereinheit vorgibt, dass zwei aufeinanderfolgende Positionen (30) des visuellen Verfolgungsprotokolls um einen Abstand (EM1, EM2, EM3, EM4) getrennt sind, der entlang des visuellen Verfolgungsprotokolls variiert.

11. Testvorrichtung (10) nach einem der Ansprüche 1 bis 10, wobei die Steuereinheit eine privilegierte senkrechte (SV) und waagrechte Verlaufsrichtung (SH) des visuellen Verfolgungsprotokolls speichert.

12. Testvorrichtung (10) nach einem der Ansprüche 1 bis 11, wobei das visuelle Verfolgungsprotokoll einer Lesestrecke gemäß derjenigen folgt, die von einem gegebenen Schreibsystem definiert wird, um die Verschiebung des Blicks der Person (1) während des Lesens gemäß dem Schreibsystem zu reproduzieren.

13. Verfahren zur Bestimmung mindestens eines optischen Gestaltungsparameters einer zur Ausstattung eines von der Person (1) ausgewählten Gestells bestimmten ophthalmischen Linse, abhängig von deren Sehverhalten, wobei das Verfahren die Testvorrichtung (10) nach einem der Ansprüche 1 bis 12 verwendet und die folgenden Schritte enthält:
a) die Person (1) wird aufgefordert, eine visuelle Aufgabe auszuführen, bei der sie das visuell ausschlaggebende Ziel (20) betrachtet, das vom Display (11) der Anzeigevorrichtung angezeigt wird und geeignet ist, den Blick der Person (1) während des Sehtests auf sich zu ziehen, wobei das Ziel (20) der Person (1), die nacheinander in eine Vielzahl gewünschter besonderer Richtungen blickt, die je einer vom Ziel (20) eingenommenen besonderen Anzeigeposition (30) zugeordnet sind, ein visuelles Verfolgungsprotokoll vorgibt, wobei das visuelle Verfolgungsprotokoll der zeitlichen Aufeinanderfolge der vom visuell ausschlaggebenden Ziel (20) eingenommenen Positionen entspricht, wobei die Positionen (30) des Ziels (20) in einem mit einem Bildaufnahmegerät verbundenen Bezugssystem vorbestimmt sind,
b) mittels des Bildaufnahmegeräts Bilder des Kopfes der das Ziel (20) betrachtenden Person (1) aufgenommen werden, wobei jedes Bild einer vorbestimmten Position (30) des Ziels (20) entspricht,
c) ausgehend von mindestens einem Teil der Bilder des Kopfes der Person (1) Positionen des Kopfes der Person (1) in einem mit dem Bildaufnahmegerät verbundenen Bezugssystem oder Blickrichtungen (DR) der Person (1) in einem mit dem Kopf der Person (1) verbundenen Bezugssystem bestimmt werden, wobei jede Position des Kopfes oder Blickrichtung der Person (1) der Position (30) des Ziels (20) zugeordnet ist, für die das entsprechende Bild des Kopfes der Person (1) aufgenommen wurde,
d) der gesuchte optische Gestaltungsparameter ausgehend von den Positionen des Kopfes oder der Blickrichtung der Person (1) abgeleitet wird.

14. Bestimmungsverfahren nach Anspruch 13, gemäß dem vor dem Schritt d) ausgehend von den bestimmten Positionen des Kopfes oder den Blickrichtungen der Person (1) die Positionen (70) des Ziels (20) in einem mit dem Kopf der Person (1) verbundenen Bezugssystem erneut ausgedrückt werden, und im Schritt d) der gesuchte optische Gestaltungsparameter ausgehend von den Positionen (70) des Ziels (20) im mit dem Kopf der Person (1) verbundenen Bezugssystem abgeleitet wird.

## Claims

1. Device (10) for testing the visual behavior of an individual (1), this device including:
- an active display (11) suitable for displaying at least one visually predominant target (20) in a plurality of positions (30) that vary over time and that are aligned in at least one row (L1, L2) or one column, and
- a unit for controlling the display (11), this unit being programmed so that the successively displayed positions (30) of the target (20) follow, over time, a visual tracking protocol
**characterized in that**:
all the successively displayed positions (30) are such that the target (20) is in the visual field of the individual, and
the visually predominant target (20) is a target that is suitable for catching the eye of said individual (1) following said target (20) during the eye test, said visual tracking protocol corresponding to the succession, over time, of positions adopted by the visually predominant target (20), said target (20) imposing a visual tracking protocol on the individual (1) who successively looks in a plurality of particular desired directions that are each associated with a particular display position (30) adopted by the target (20) .

2. Testing device (10) according to Claim 1, wherein said positions (30) of the target (20) are aligned in at least two rows (L1, L2) or two columns that are substantially parallel.

3. Testing device (10) according to either of Claims 1 and 2, wherein said plurality comprises, in each row or column, at least three aligned positions (35, 36, 37) of said target (20).

4. Testing device (10) according to one of Claims 1 to 3, wherein the controlling unit makes it so that, in each position (30) of said visual tracking protocol, the target (20) is displayed for a predetermined duration.

5. Testing device (10) according to Claim 4, wherein said predetermined duration is comprised between 50 milliseconds and 1 second.

6. Testing device (10) according to Claim 4 or 5, wherein said target (20) remains stationary for said predetermined duration.

7. Testing device (10) according to one of Claims 1 to 6, wherein the controlling unit makes it so that there is a predetermined lag between the display of the target (20) in two successive positions (30) of the visual tracking protocol.

8. Testing device (10) according to Claim 7, wherein said predetermined lag varies over the course of the visual tracking protocol.

9. Testing device (10) according to one of Claims 1 to 8, wherein said controlling unit makes it so that two successive positions (30) of the visual tracking protocol are separated by a distance (EM1, EM2, EM3, EM4) smaller than 10 centimeters.

10. Testing device (10) according to one of Claims 1 to 9, wherein said controlling unit makes it so that two successive positions (30) of the visual tracking protocol are separated by a distance (EM1, EM2, EM3, EM4) that varies throughout the visual tracking protocol.

11. Testing device (10) according to one of Claims 1 to 10, wherein said controlling unit stores a favored vertical direction of travel (SV) and a favored horizontal direction of travel (SH) of the visual tracking protocol in memory.

12. Testing device (10) according to one of Claims 1 to 11, wherein the visual tracking protocol follows a reading trajectory which accords with that defined by a given writing system, so as to reproduce the displacement of the gaze of the individual (1) while reading in accordance with the writing system.

13. Method for determining at least one optical parameter for designing an ophthalmic lens intended to be mounted in a frame chosen by the individual (1), depending on the visual behavior of the latter, said method using the testing device (10) according to one of Claims 1 to 12 and comprising the following steps:
a) the individual (1) is asked to perform a visual task in which they look at the visually predominant target (20) that is displayed by said display (11) of the display device and that is suitable for catching the eye of said individual (1) during the eye test, said target (20) imposing a visual tracking protocol on the individual (1) who successively looks in a plurality of particular desired directions that are each associated with a particular display position (30) adopted by the target (20), said visual tracking protocol corresponding to the succession, over time, of positions adopted by the visually predominant target (20), the positions (30) of said target (20) being predetermined in a frame of reference attached to an image-capturing apparatus,
b) images of the head of the individual (1) looking at said target (20) are captured by means of said image-capturing apparatus, each image corresponding to a predetermined position (30) of said target (20),
c) on the basis of at least some of the images of the head of the individual (1), positions of the head of the individual (1) in a frame of reference attached to said image-capturing apparatus or gaze directions (DR) of the individual (1) in a frame of reference attached to the head of the individual (1) are determined, each position of the head or gaze direction of the individual (1) being associated with the position (30) of said target (20) for which the corresponding image of the head of the individual (1) was captured,
d) said sought-after optical design parameter is deduced from said positions of the head or gaze direction of the individual (1).

14. Determining method according to Claim 13, wherein, prior to step d), the positions (70) of said target (20) are re-expressed, on the basis of said determined positions of the head or the gaze directions of the individual (1), in a frame of reference attached to the head of the individual (1), and, in step d), said sought-after optical design parameter is deduced from said positions (70) of the target (20) in the frame of reference attached to the head of the individual (1).
